# EUROPÄISCHE PATENTANMELDUNG

(11) **EP 2 581 031 A1**
(43) Veröffentlichungstag der Anmeldung: **17.04.2013**
(21) Anmeldenummer: 12188517.2
(22) Anmeldetag: 15.10.2012
(51) Int. Cl.: A61B 1/005

(54) **Abwinkelungsvorrichtung**

(30) Priorität: 13.10.2011 AT 14932011
(71) Anmelder: Klaffenböck, Johann, 5350 Strobl (AT); Klaffenböck, Lukas, 8010 Graz (AT); Mair, Julian, 81543 München (DE)
(72) Erfinder: Klaffenböck, Johann, 5350 Strobl (AT); Klaffenböck, Lukas, 8010 Graz (AT); Mair, Julian, 81543 München (DE)
(74) Vertreter: Babeluk, Michael

(57) **Zusammenfassung**

Die Erfindung betrifft eine Abwinkelungsvorrichtung (100), insbesondere für ein chirurgisches Instrument, mit einem im wesentlichen rohrförmigen, aus zumindest zwei, vorzugsweise aus einer Vielzahl von im wesentlichen ringförmigen Segmenten (10) gebildeten Grundkörper (101) mit einem Innenraum (103), der eine im wesentlichen zentral verlaufende Längsachse (A) aufweist, wobei zumindest ein Segment (10) gegenüber zumindest einem benachbarten Segment (10) um eine Schwenkachse (B), die ungleich der Längsachse (A) des Grundkörpers (101) ist, aus einer ersten Position in eine zweite Position verschwenkbar ist, und das zumindest eine Segment (10) über eine in sich geschlossene Fuge (102) von zumindest einem benachbarten Segment (10) beabstandet ist, wobei das Segment (10) unverlierbar an dem benachbarten Segment (10) angeordnet ist. Eine robuste Ausführung, die eine starke Abwinkelung ermöglicht, wird dadurch erreicht, dass die Vorsprünge (12) jeweils über eine Materialbrücke (16) mit dem Grundkörper (17) des Segments (10, 10') verbunden sind, die vollständig innerhalb eines Quadranten (18) liegt, der ausgehend vom Mittelpunkt (M) des Vorsprungs (12) einerseits durch eine Parallele (D) zur Längsachse (A) und einer Normalen (N) auf diese Parallele (D) definiert ist.

## Beschreibung

Die Erfindung betrifft eine Abwinkelungsvorrichtung für ein chirurgisches Instrument gemäß dem Oberbegriff von Patentanspruch 1. Insbesondere betrifft die Erfindung eine Abwinkelungsvorrichtung mit einem im wesentlichen rohrförmigen, aus zumindest zwei, vorzugsweise aus einer Vielzahl von im wesentlichen ringförmigen Segmenten gebildeten mit einem Innenraum, der eine im wesentlichen zentral verlaufende Längsachse aufweist, wobei zumindest ein Segment gegenüber zumindest einem benachbarten Segment um eine Schwenkachse, die ungleich der Längsachse des Grundkörpers ist, aus einer ersten Position in zumindest eine zweite Position verschwenkbar ist, wobei das zumindest eine Segment unverlierbar an dem benachbarten Segment angeordnet ist, indem das zumindest eine Segment über zumindest zwei diametral zueinander angeordnete Gelenksbereiche verschwenkbar ist, die durch teilweise kreisförmige Vorsprünge gebildet sind, die von einem Grundkörper des Segments vorstehen und die jeweils in einer Aufnahme eines benachbarten Segmentes gelagert sind.

Die Erfindung betrifft auch ein medizinisches Instrument mit einer derartigen Abwinkelungsvorrichtung und ein Verfahren zu dessen Herstellung.

In der Medizin, insbesondere in der minimal-invasiven Chirurgie kommen chirurgische Instrumente zum Einsatz, die über einen langen Schaft verfügen, über den die für die Operation notwendigen Gerätschaften in den Körper des Patienten, beispielsweise über Körperöffnungen wie Mund und Speiseröhre, eingeführt werden. Dieser Schaft ist üblicherweise krümmbar ausgebildet, um an die zu behandelnde Stelle innerhalb des Körpers des Patienten zu gelangen. Um diese Abwinkelung des Schaftes zu erreichen, sind zahlreiche Vorrichtungen bekannt geworden. So beschreibt die DE 44 14 810 C1 ein Endoskoprohrsystem mit einem biegbaren Abschnitt, der aus gelenkig oder federelastisch miteinander verbundenen Einzelgliedern aufgebaut ist. Ähnliche Vorrichtungen sind auch in der US 2007/0038230 A1, in der DE 199 28 272 A1 oder in der DE 101 13 713 C1 offenbart.

Diesem Stand der Technik kann allgemein entnommen werden, dass bei einer ersten Gruppe von Abwinkelungsvorrichtungen diese aus Einzelgliedern gefertigt sind, die teilweise einen relativ komplizierten Aufbau aufweisen. Nachteilig an diesem Typus ist, dass sich diese Einzelglieder im Gebrauch aufgrund von Belastung voneinander lösen können, sodass die Bedienung der Abwinkelungsvorrichtung und in der Folge die des (chirurgischen) Instrumentes unmöglich wird, was Komplikationen im Zuge einer Operation hervorrufen kann.

Bei einer zweiten bekannt gewordenen Gruppe von Abwinkelungsvorrichtungen werden Einschnitte in ein Rohr vorgenommen, um auf diese Weise eine federelastische Abwinkelung zu erzielen, wobei hier aufgrund der Einstückigkeit die Problematik der oben beschriebenen fehlenden Bedienbarkeit deutlich reduziert wird. Nachteilig an derartigen Aufbauten ist jedoch, dass bedingt durch das Material nur geringe Krümmungsradien erzielt werden können. Beispielsweise wird bei der laparoskopischen Behandlung von Refluxösophagitis (GERD), einer entzündlichen Erkrankung der Speiseröhre, die Verbindungsstelle zwischen Speiseröhre und Magen mittels einer Naht verengt, wobei das hierfür eingesetzte chirurgische Instrument einen sehr engen Abwinkelungsradius aufweisen muss, um die zu vernähende Stelle zu erreichen. Diese Vorrichtungen sind hierfür nur bedingt bis gar nicht geeignet.

Aus der EP 0 764 423 A ist ein abwinkelbares Rohr bekannt, das als Abwinkelungsvorrichtung im obigen Sinn einsetzbar ist. Mit diesem Rohr kann ein Teil der oben dargestellten Probleme gelöst werden, wobei insbesondere der robuste Aufbau und die leichte Herstellbarkeit hervorzuheben sind. In einer Ausführungsvariante werden durch kreisförmige Vorsprünge Gelenke gebildet, um die die einzelnen Segmente gegeneinander verschwenkbar sind. Weitere Vorsprünge führen die Segmente in einer Richtung parallel zur Schwenkachse und begrenzen den Schwenkwinkel. Nachteilig bei dieser Vorrichtung ist, dass der Schwenkwinkel begrenzt und für manche Anwendungen nicht ausreichend ist. Darüber hinaus treten im verschwenkten Zustand die weiteren Vorsprünge ins Innere des Rohres ein, was zu einer Beschädigung von Strukturen führen kann, die im Inneren des Rohres geführt sind, wie etwa optische Fasern oder dergleichen. Ein weiterer Nachteil besteht darin, dass die Übertragung von Zugkräften in Axialrichtung nur begrenzt möglich ist, weil bei Anwendung zu großer Kräfte die kreisförmigen Vorsprünge aus ihren Aufnahmen herausgezogen werden können, wodurch diese verformt und letztlich zerstört werden.

Es ist daher Aufgabe der Erfindung, eine Abwinkelungsvorrichtung bereit zustellen, die die obengenannten Nachteile des Stands der Technik beseitigt, und die insbesondere einen engen Abwinkelungsradius ermöglicht, wobei die Vorrichtung kostengünstig und auf einfache Weise hergestellt werden kann. Auch bei sehr enger Abwinkelung soll der Innenraum weitgehend frei von eindringenden Bauteilen bleiben. Eine weitere Aufgabe besteht darin, dass eine erhöhte mechanische Stabilität erzielt wird, und zwar insbesondere gegenüber Zugkräften.

Diese Aufgabe wird erfindungsgemäß durch eine Abwinkelungsvorrichtung gemäß Patentanspruch 1 gelöst. Insbesondere ist vorgesehen, dass die Vorsprünge jeweils über eine Materialbrücke mit dem Grundkörper des Segments verbunden sind, die vollständig innerhalb eines Quadranten liegt, der ausgehend vom Mittelpunkt des Vorsprungs durch eine Parallele zur Längsachse und einer Normalen auf diese Parallele definiert ist, und dass das zumindest eine Segment über eine in sich geschlossene Fuge von zumindest einem benachbarten Segment beabstandet ist, wobei das Segment unverlierbar an dem benachbarten Segment angeordnet ist.

Jedes Segment ist hierbei einerseits einstückig gefertigt, sodass es zu keiner Beeinträchtigung der Verschwenkung aufgrund einer mangelnden Flexibilität des Materials kommt, während andererseits der Verlauf der Fuge derart gewählt ist, dass das Segment jeweils unverlierbar an dem benachbarten Segment angeordnet ist. Zur Befestigung des Segments an einem benachbarten Segment sind erfindungsgemäß keinerlei zusätzlichen Vorrichtungen oder Verbindungselemente notwendig.

Unter "unverlierbar" wird im Rahmen dieser Offenbarung der Umstand verstanden, dass ein erstes Segment von einem benachbarten zweiten Segment nur unter Zerstörung der Vorrichtung entfernt werden kann. Unter "in sich geschlossene Fuge" wird im Rahmen dieser Offenbarung ein materialfreier Bereich zwischen zwei benachbarten Segmenten verstanden, der die beiden benachbarten Segmente voneinander vollständig trennt.

Ein wesentlicher Aspekt der vorliegenden Erfindung besteht darin, dass die kreisförmigen Vorsprünge nicht symmetrisch in Axialrichtung vorragen, sondern schräg, also aus der Axialrichtung weg geneigt angeordnet sind. Dadurch kann einerseits der Schwenkwinkel wesentlich vergrößert werden, so dass eine besonders enge Abwinkelung erreichbar ist. Andererseits wird dadurch erreicht, dass die Vorsprünge in Axialrichtung von Teilen der Aufnahme des folgenden Segments hintergriffen werden, so dass eine besonders hohe Übertragung von Zugkräften ermöglicht wird.

Dieser enge Verschwenkungsradius wird weiter dadurch unterstützt, dass das zumindest eine Segment zwischen den Gelenksbereichen eine größere axiale Erstreckung als im Gelenksbereich selbst aufweist. Damit wird zwischen zwei miteinander verbundenen Segmenten eine zwischen den Gelenksbereichen liegende Verbreiterung der Fuge erhalten, die die Abwinkelung der erfindungsgemäßen Abwinkelungsvorrichtung in einem hohen Maß erlaubt. Hierbei ist diese im wesentlichen keilförmige Verbreiterung der Fuge in der ersten Position des Segmentes in einem ersten Abschnitt zwischen den beiden Gelenken angeordnet, während bei Verschwenken des Segmentes in die zweite Position diese Verbreiterung geschlossen und ein der ersten Verbreiterung diametral gegenüberliegender zweiter Abschnitt der Fuge entsprechend verbreitert wird.

Die erfindungsgemäße Eigenschaft der Abwinkelungsvorrichtung, wonach die jeweiligen Segmente unverlierbar an den benachbarten Segmenten angeordnet sind, wird in einer besonders bevorzugten Ausführung der Erfindung dadurch erreicht, dass das zumindest eine Segment in einem ersten Abschnitt des Grundkörpers zwischen den beiden Gelenksbereichen einen sich in Axialrichtung erstreckenden, im wesentlichen rechteckigen ersten Fortsatz und dem ersten Fortsatz gegenüberliegend eine erste Ausnehmung aufweist, die zur Aufnahme des ersten Fortsatzes eines benachbarten Segmentes ausgebildet ist.

Es ist besonders bevorzugt, dass das zumindest eine Segment in einem weiteren Abschnitt des Grundkörpers zwischen den beiden Gelenksbereichen, der dem ersten Abschnitt in Umfangsrichtung gegenüberliegt, eine im Wesentlichen glatte, keilförmige Ausnehmung aufweist. Die keilförmige Ausnehmung ermöglicht eine besonders große Abwinkelung, wobei die Abwesenheit von Vorsprüngen oder dergleichen das Eindringen von Teilen des Rohres in das Lumen verhindert.

Eine besonders begünstigte Ausführungsvariante der Erfindung sieht vor, dass ein hakenförmiger Vorsprung eines Segments einen teilweise kreisförmigen Vorsprung des benachbarten Segments mit einem Umfangswinkel von mindestens 90° umgreift. Insbesondere ragt der hakenförmige Vorsprung dabei in der gestreckten Stellung der Abwinkelungsvorrichtung über die Parallele zur Längsachse, die durch dem Mittelpunkt des teilweise kreisförmigen Vorsprungs geht, hinaus, d.h. er hintergreift ihn. Dadurch wird eine besondere Zugfestigkeit erreicht.

In einer weiteren Ausführung der Erfindung sind die ersten und zweiten Fortsätze entlang einer Linie angeordnet sind, die parallel zu der Längsachse des Grundkörpers verläuft. Hierbei erfolgt bei Abknickung der erfindungsgemäßen Abwinkelungsvorrichtung die Verschwenkung der einzelnen Segmente um Schwenkachsen, die jeweils parallel zueinander angeordnet sind, sodass die Verschwenkung der einzelnen Segmente jeweils in die gleiche Richtung erfolgt.

Alternativ hierzu ist in einer weiteren Ausführung vorgesehen, dass die ersten und zweiten Fortsätze in Umfangsrichtung des Grundkörpers versetzt zueinander angeordnet sind, sodass bei Verschwenken der einzelnen Segmente eine Verkrümmung bzw. Verdrehung der Abwinkelungsvorrichtung erhalten wird. Diese Verkrümmung wird durch den versetzten Aufbau der einzelnen Schwenkachsen der einzelnen Segmente zueinander erzielt.

In einer weiteren bevorzugten Ausführung der Erfindung weist das zumindest eine Segment vier Gelenksbereiche auf, die paarweise diametral zueinander angeordnet sind, wobei ein erstes Gelenksbereichpaar über jeweils zwei Aufnahmen zur Aufnahme von Fortsätzen eines benachbarten Segmentes verfügt, während das zweite Gelenksbereichpaar über jeweils zwei Fortsätze verfügt, die in die jeweiligen Aufnahmen eines benachbarten Segmentes zumindest teilweise eingreifen. Bei dieser Ausführung können die einzelnen Segmente aus einer Mittelstellung in zwei unterschiedliche Richtungen verschwenkt werden, was eine besonders flexibles Verhalten der Abwinkelungsvorrichtung bei gleichzeitig kleinen Abwinkelungsradien zur Folge hat.

In einer weiteren besonders bevorzugten Ausführung der Erfindung ist vorgesehen, dass jedes Segment unabhängig von den benachbarten Segmenten verschwenkbar ist, wodurch eine verbesserte Anpassung der Abwinkelungsvorrichtung und in der Folge beispielsweise des chirurgischen Instruments an die jeweiligen Anforderungen erreicht wird. Hierbei sind die einzelnen Segmente über zumindest eine hydraulische, pneumatische und/oder mechanische Einrichtung bewegbar sind.

In einer weiteren vorteilhaften Ausführung der Erfindung ist vorgesehen, dass das zumindest eine Segment zumindest eine, vorzugsweise zwei oder mehrere Aufnahmen für Führungselemente, insbesondere Zugseile oder Bowdenzüge aufweist. Diese Aufnahmen sind in einer bevorzugten Ausführung der Erfindung als Bohrungen z.B. für die Aufnahme von Halteelemente oder als Laschen ausgeführt, die in den Innenraum des Grundkörpers hineindrückbar sind, durch die die Zugseile oder Bowdenzüge geführt werden.

Die Aufgabe wird des Weiteren durch ein medizinisches Instrument mit einem entlang seiner Längsachse krümmbaren länglichen Element gelöst, wobei diese eine Abwinkelungsvorrichtung gemäß der Erfindung aufweist. Hierbei ist diese Abwinkelungsvorrichtung besonders bevorzugt über zumindest ein oder zwei Führungselemente, insbesondere Zugseile oder Bowdenzüge, oder alternativ hierzu über ein Hydrauliksystem bedienbar. Ein derartiges medizinisches Instrument kann insbesondere für Operationen eingesetzt werden, bei welchen eine Krümmung des länglichen Elements um sehr enge Radien benötigt wird.

Schließlich wird die Erfindung zusätzlich durch ein Verfahren zur Herstellung einer Abwinkelungsvorrichtung dadurch gelöst, dass ein Rohr zumindest abschnittsweise in Segmente zerteilt wird, wobei die Segmente voneinander abgetrennt, unlösbar voneinander und gleichzeitig ineinander eingreifend unter Bildung einer in sich geschlossenen Fuge zugeschnitten werden.

In einer Ausführungsvariante des erfindungsgemäßen Verfahrens verbleiben während des Schneidens kleine Stege, die die Fuge unterbrechen und in einem weiteren Fertigungsschritt der Abwinkelungsvorrichtung, jedoch spätestens vor der erstmaligen Benutzung der Abwinkelungsvorrichtung aufgebrochen werden.

Besonders bevorzugt wird hierbei die Zerteilung des Rohres mittels Laserschneidtechnologie durchgeführt, das besonders enge und präzise Schnittradien erlaubt. Alternativ hierzu erfolgt die Herstellung des Grundkörpers mittels Wasserstrahlschneiden, Plasmaschneiden, Erosion, 3D-Printing oder Gießen.

Im Folgenden wird anhand eines nicht einschränkenden Ausführungsbeispiels mit zugehören Figuren die Erfindung näher erläutert. Darin zeigen
- Fig. 1: eine Schrägansicht der erfindungsgemäßen Abwinkelungsvorrichtung in einer ersten, gestreckten Position;
- Fig. 2: eine Seitenansicht der Abwinkelungsvorrichtung in einer zweiten, gekrümmten Position;
- Fig. 3: eine Schrägansicht der Abwinkelungsvorrichtung in einer zweiten, gekrümmten Position;
- Fig. 4: eine Abwicklungsdarstellung eines Segmentes der erfindungsgemäßen Abwinkelungsvorrichtung gemäß Fig. 1;
- Fig. 5: eine Seitenansicht einer zweiten Ausführungsform der erfindungsgemäßen Abwinkelungsvorrichtung in der gestreckten Position;
- Fig. 6: eine Seitenansicht einer dritten Ausführungsform der erfindungsgemäßen Abwinkelungsvorrichtung in der gestreckten Position;
- Fig. 7 bis 9: unterschiedliche perspektivische Ansichten der Abwinkelungsvorrichtung aus Fig. 6 in gekrümmter Position;
- Fig. 10: eine Schrägansicht eines Teils der Abwinkelungsvorrichtung in einer vierten Ausführungsvariante;
- Fig. 11: eine Schrägansicht eines ersten Segments der Abwinkelungsvorrichtung von Fig. 10; und
- Fig. 12 bis 14: unterschiedliche perspektivische Ansichten der Abwinkelungsvorrichtung von Fig. 10 in gekrümmter Position.

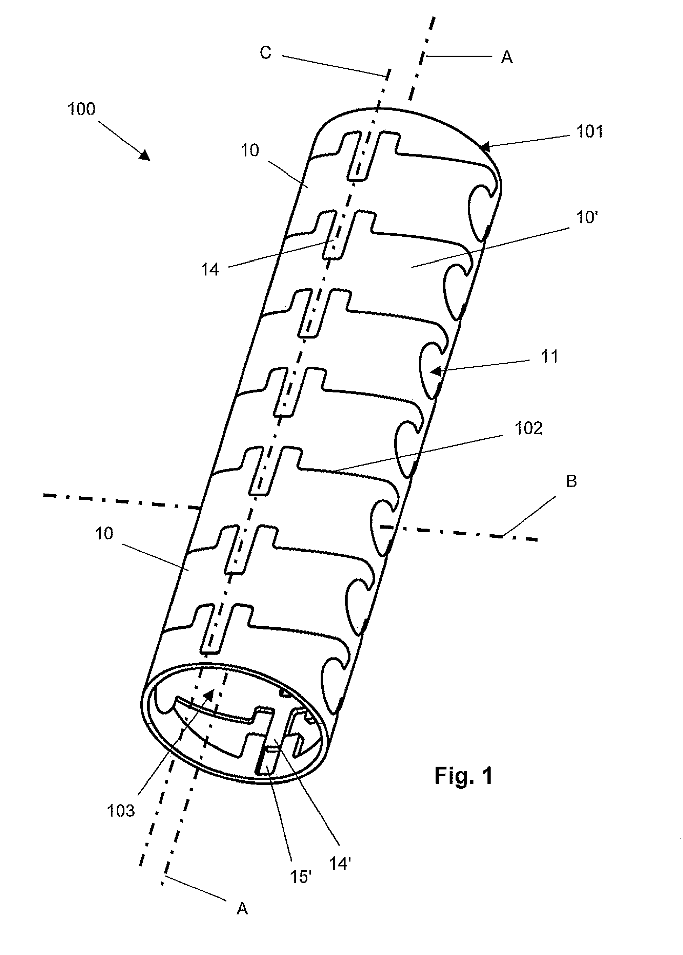

Die Fig. 1 zeigt eine erste Ausführung der erfindungsgemäßen Abwinkelungsvorrichtung 100 mit einem Grundkörper 101, der aus einer Mehrzahl von Segmenten 10, 10' aufgebaut ist, wobei die Segmente 10, 10' durch eine Fuge 102 voneinander abgegrenzt sind. Die Segmente 10, 10' haben einen im wesentlichen ringförmigen Körper und sind über gelenkige Verbindungen, die den Gelenksbereich 11 bilden, mit benachbarten Segmenten 10, 10' verbunden. Durch den ringförmigen Körper der Segmente 10, 10' wird im Grundkörper 101 der Abwinkelungsvorrichtung 100 ein Innenraum 103 mit einer Längsachse A gebildet. Die Dimensionen des Innenraums 103 sind beispielsweise derart gewählt, dass Steuerungselemente für Instrumente und/oder Endoskope durch den Grundkörper hindurchgeführt werden können.

Die Schwenkachse B der Segmente 10, 10', die durch zwei diametral am Grundkörper 101 angeordnete gelenkige Verbindungen 11 verläuft, steht normal auf die Längsachse A des Grundkörpers 101.

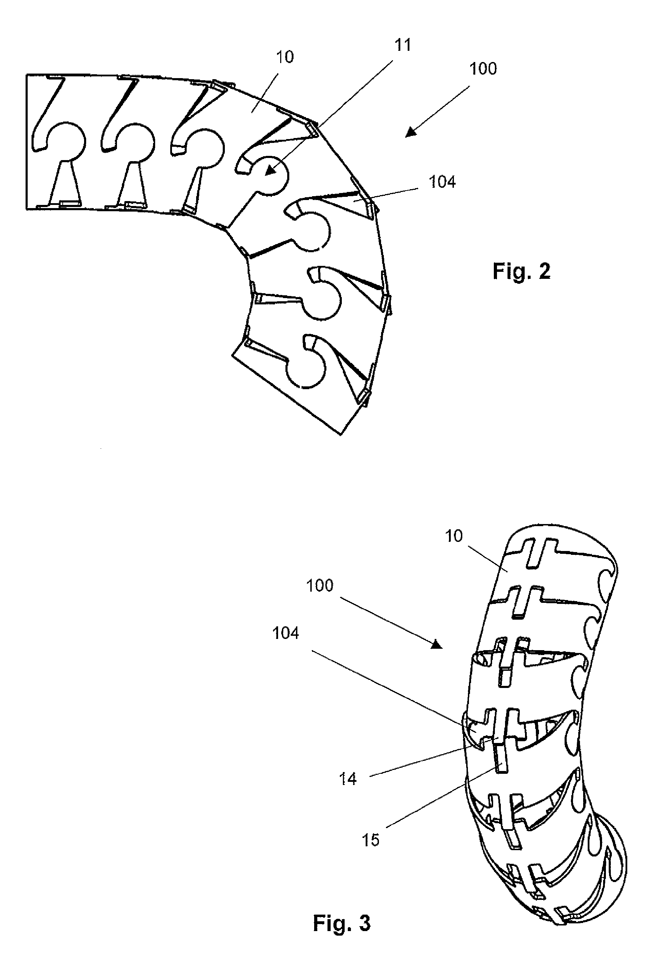

In den Fig. 2 und 3 ist die Abwinkelungsvorrichtung 100 in gekrümmter Position dargestellt, wobei deutlich ersichtlich ist, dass die Segmente 10, 10' je nach Krümmungsradius unterschiedlich stark um die gelenkige Verbindung 11 und damit um die Schwenkachse B verschwenkt sind.

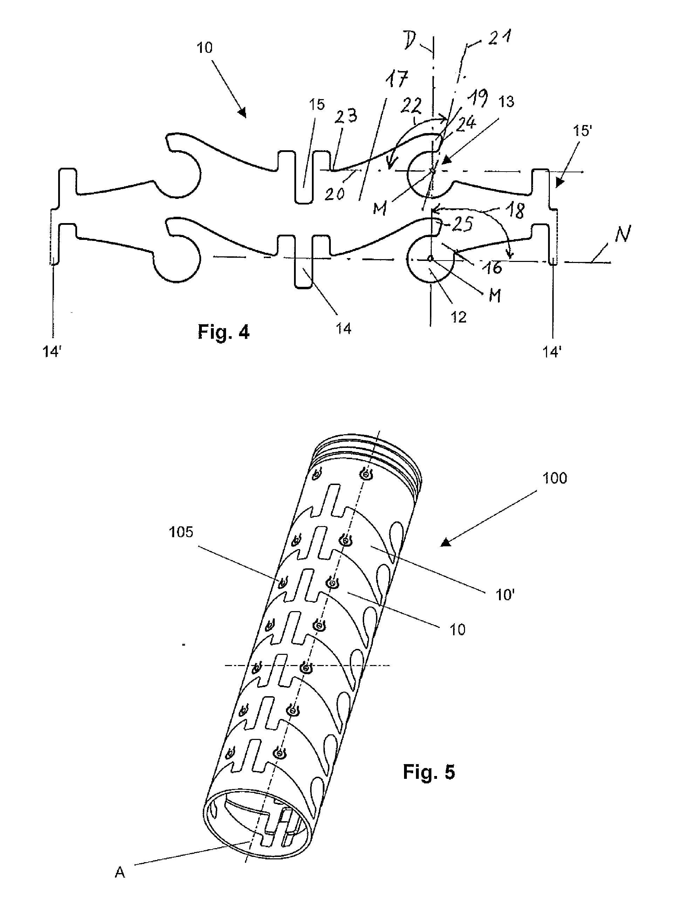

Die gelenkige Verbindung 11 wird durch einen Vorsprung 12 des Segmentes 10 gebildet, wobei der teilweise kreisförmige Vorsprung 12 in eine Aufnahme 13 des benachbarten Segmentes 10 formschlüssig eingreift und darin verdrehbar angeordnet ist (Fig. 4). Die Verdrehbarkeit des Vorsprunges 12 in der Aufnahme 13 bewirkt die Verschwenkbarkeit des Segmentes 10 um die Schwenkachse B, wobei der Grad der Verschwenkbarkeit durch die im Wesentlichen kreisförmig hinterschnittene Form des teilweise kreisförmigen Vorsprungs 12 bedingt ist. Um eine Verschiebung des Segmentes 10 entlang der Verschwenkachse B innerhalb des Grundkörpers 101 zu vermeiden, weist das Segment einen Fortsatz 14 auf, der je nach Verschwenkungsgrad des Segmentes 10 zumindest teilweise in eine Ausnehmung 15 eines benachbarten Segmentes 10 eingreift.

Es wird nun unter Bezugnahme auf Fig. 4 die Geometrie eines erfindungsgemäßen Segments 10 erklärt. Der teilweise kreisförmige Vorsprung 12 ist über eine Materialbrücke 16 an dem Grundkörper 17 des Segments 10 einstückig angeformt. Diese Materialbrücke 16 liegt vollständig innerhalb eines Quadranten 18, der durch eine Parallele D zur Längsachse A und einer Normalen N dazu gebildet ist, wobei die Parallele D und die Normale N durch den Mittelpunkt M des teilweise kreisförmigen Vorsprungs 12 gehen.

In Entsprechung dazu ist ein hakenförmiger Vorsprung 19 des Segments 10 vorgesehen, der die Aufnahme 13 teilweise umschließt und diese mit einem Umfangswinkel 22 von mindestens 90° umgreift, hier mit etwa 110°. Der Umfangswinkel 22 wird durch zwei gedachte Geraden gebildet, nämlich eine erste Gerade 20, die sich vom Mittelpunkt M ausgehend bei 23 innen tangential an die Kontur des Segments 10 anlegt und eine zweite Gerade 21, die den Mittelpunkt M und die Spitze 24 des hakenförmigen Vorsprungs 19 miteinander verbindet.

Dem hakenförmigen Vorsprung 19 entspricht eine hakenförmige Ausnehmung 25, die erfindungsgemäß die Parallele D zur Längsachse A überquert und damit in der gestreckten Stellung ein Hintergreifen durch den hakenförmigen Vorsprung 19 ermöglicht.

Die einzelnen Segmente 10, 10' innerhalb des Grundkörpers 101 sind durch die Fuge 102 voneinander getrennt. Diese Fuge 102 verbreitert sich in den Bereichen zwischen Vorsprung 12 und Fortsatz 14 des Segmentes 10, sobald der Grundkörper 101 gekrümmt wird. Hierbei bewegt sich der Fortsatz 14 aus der Ausnehmung 15 des benachbarten Segments 10 heraus, wobei dieses Herausbewegen nicht zur Gänze erfolgt, um ein Verschieben der Segmente 10, 10' entlang der Schwenkachse B zu verhindern. Gleichzeitig bewegt sich ein zweiter, dem ersten Fortsatz 14 diametral gegenüberliegender Fortsatz 14' in eine zweite Ausnehmung 15' eines benachbarten Segmentes 10 hinein.

In der gestreckten Position der Abwinkelungsvorrichtung 100 (Fig. 1) sind hingegen die Innen- sowie Außenwände des Grundkörpers 101 völlig eben, wobei der erste Fortsatz 14 zur Gänze in der Ausnehmung 15 des benachbarten Segments 10' aufgenommen ist, während der zweite Fortsatz 14' teilweise in der zweiten Ausnehmung 15' hineinragt, und die Fuge 102 im Bereich zwischen zweiten Fortsatz 14' und der gelenkigen Verbindung 11 eine im Wesentlichen keilförmige Verbreiterung 104 aufweist.

Bei der in der Fig. 5 dargestellten Ausführung der Erfindung ist am Grundkörper 101 zu beiden Seiten der Ausnehmung 15 jeweils eine Lasche 105 angeordnet, die in der Gebrauchslage jeweils in den Innenraum 103 des Grundkörpers 101 hineinragen und der Aufnahme eines Zugmittels, beispielsweise Bowdenzüge dienen.

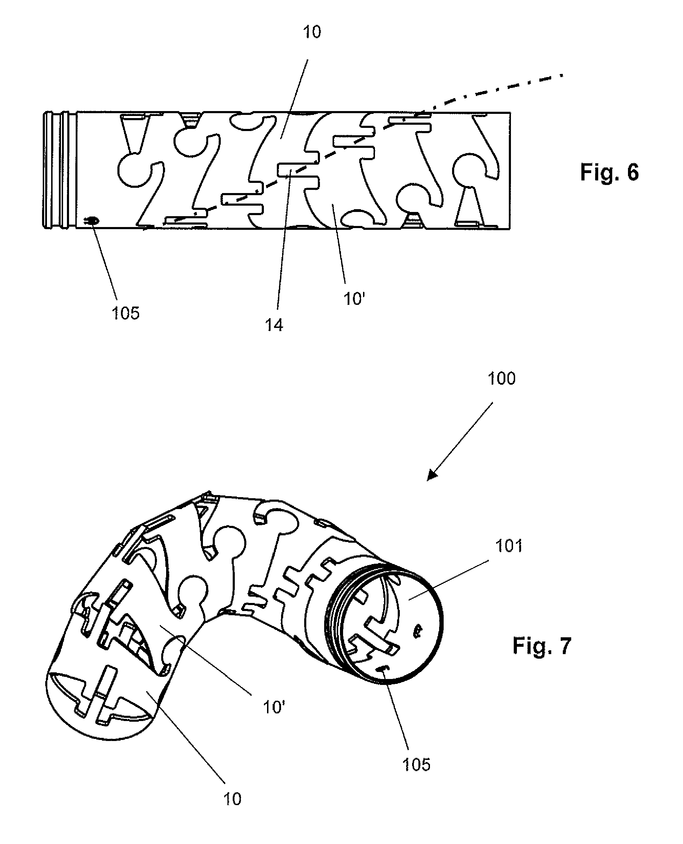

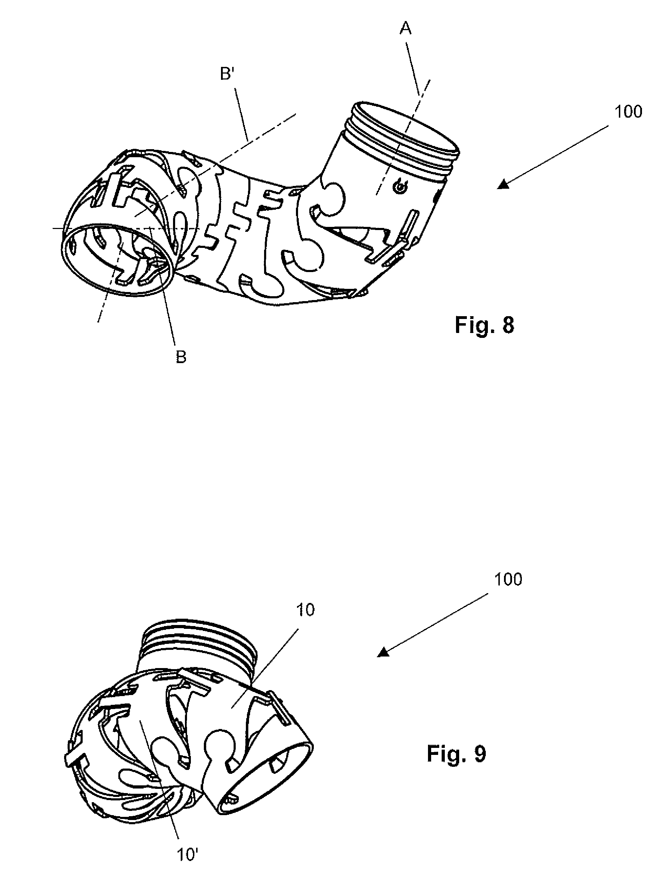

In den Fig. 6 bis Fig. 9 ist eine weitere bevorzugte Ausführung der erfindungsgemäßen Abwinkelungsvorrichtung 100 dargestellt, die ebenfalls aus einer Vielzahl von Segmenten 10, 10', die einstückig ausgebildet und über eine Fuge 102 unverlierbar miteinander in Verbindung stehen, aufgebaut ist. Bei dieser Ausführung sind jedoch die Fortsätze 14, 14' einzelnen Segmente 10, 10' versetzt zueinander entlang einer Linie C angeordnet, wodurch die Linie C eine Spirale um die Längsachse A des Grundkörpers 101 bildet. Dadurch sind auch die einzelnen Schwenkachsen B, B' der einzelnen Segmentes 10, 10' verdreht zueinander angeordnet, sodass bei Abwinkelung der erfindungsgemäßen Abwinkelungsvorrichtung 100 diese ineinander verdrehbar bzw. krümmbar ist. Damit können besonders enge Krümmungsradien erzielt und schwer zu erreichende Stellen, beispielsweise im Körperinneren eines Patienten mithilfe der Abwinkelungsvorrichtung 100 gezielt erreicht werden. Im Gegensatz hierzu verläuft die Linie C in der erfindungsgemäßen Ausführung 100 gemäß der Fig. 1 parallel zu der Längsachse A des Grundkörpers 101.

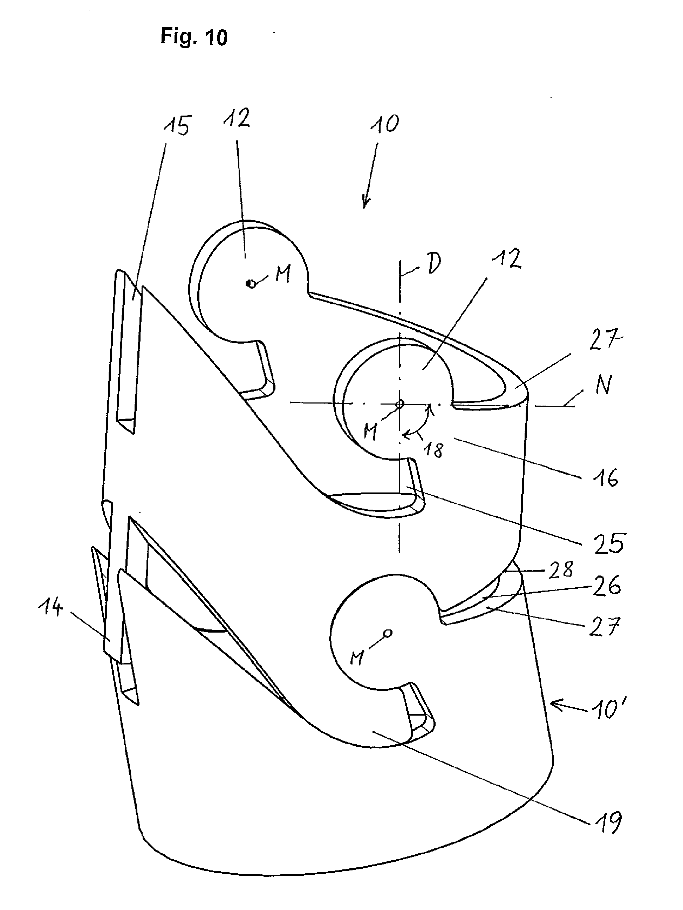

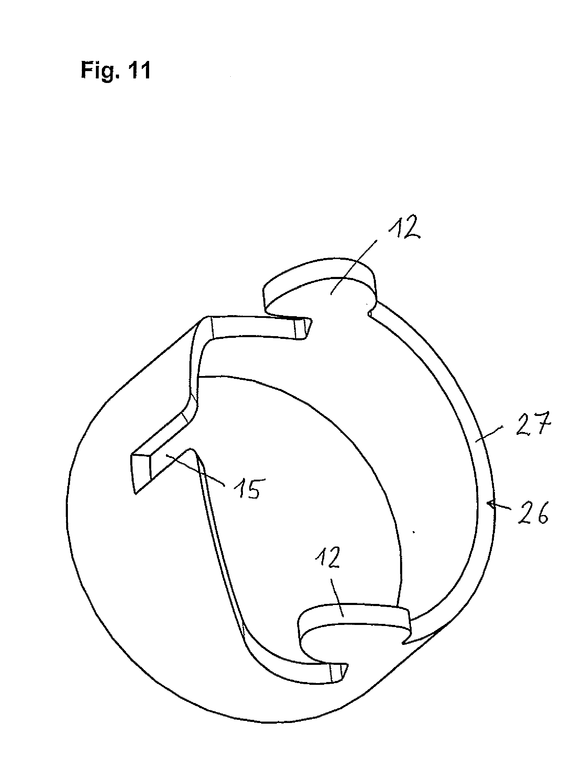

Die in der Fig. 10 bis 14 dargestellte Ausführung der erfindungsgemäßen Abwinkelungsvorrichtung 100 unterscheidet sich von den vorbeschriebenen erfindungsgemäßen Ausführungsformen dadurch, dass das einzelne Segment 10 nur einen einzigen Fortsatz 14 aufweist, der in eine entsprechende Ausnehmung 15 des benachbarten Segments 10' eingreift. Radial gegenüberliegend ist eine im Wesentlichen glatte, keilförmige Ausnehmung 26 gebildet, die frei von Vorsprüngen ist. Die Ausnehmung 26 ist durch entsprechend gekrümmte bogenförmige Kanten 27, 28 der Segmente 10, 10' gebildet, die bei maximaler Krümmung der Abwinkelungsvorrichtung aneinander anliegen. Die Kanten 27, 28 sind im Wesentlichen durch Schnitte von Ebenen gebildet, die durch die beiden Mittelpunkte M der Vorsprünge 12 hindurchgehen und gegenüber der Längsachse A einander entgegengesetzt geneigt sind.

Das untere Segment 10' ist ein erstes Segment am distalen Ende der eigentlichen Abwinkelungsvorrichtung 100.

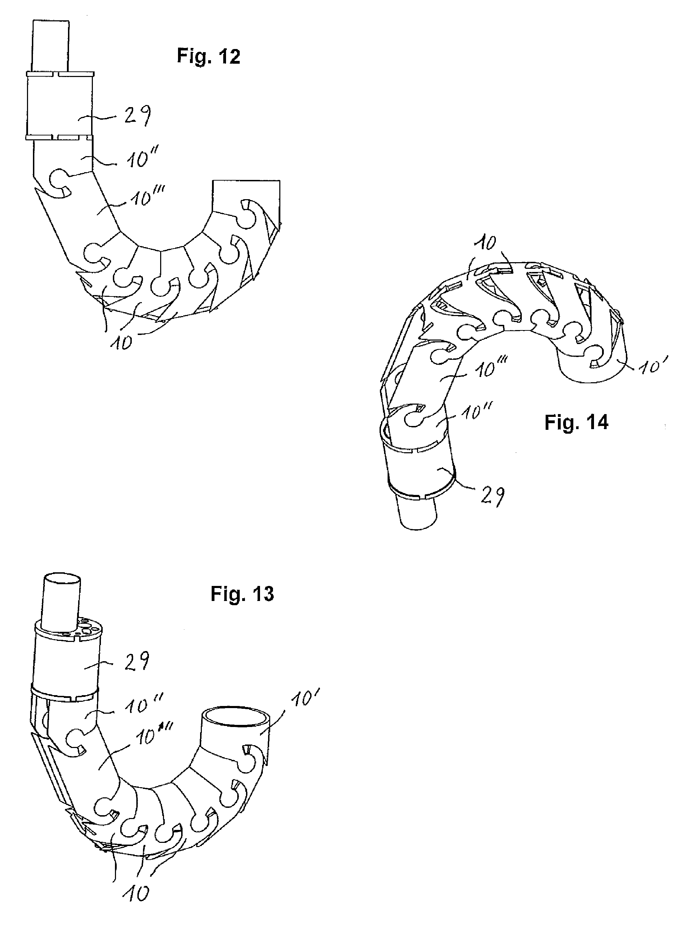

In den Fig. 12 bis Fig. 14 wird eine Abwinkelungsvorrichtung 100 in verschiedenen Ansichten dargestellt, die wie oben dargestellt aufgebaut ist.

Ein Anschlussstück 29 ist dazu vorgesehen, an einem nicht näher dargestellten chirurgischen Instrument befestigt zu werden, das dazu bestimmt ist, in eine Körperöffnung eingeführt zu werden. Ein Übergangssegment 10", das etwas länger und mit einer seitlichen Öffnung ausgebildet ist, dient zur Befestigung eines Spezialsegments 10"', das dazu dient, ein hier nicht dargestelltes Einzelinstrument, wie etwa ein Endoskop, aus dem Rohr seitlich herauszuführen. An dieses Spezialsegment 10"' schließen nunmehr mehrere Segmente 10 an.

Aus den Darstellungen ist ersichtlich, dass diese Ausführungsvariante der Abwinkelungsvorrichtung 100 nur eine einzige Abwinkelungsrichtung aufweist, die allerdings eine besonders enge Abwinkelung ermöglicht.

Die in den Ausführungsbeispielen gezeigten Ausnehmungen und Aufnahmen bzw. die Ausgestaltung der zwischen den Segmenten 10 befindlichen Fuge ist nicht einschränkend zu betrachten. Selbstverständlich kann insbesondere die Ausgestaltung der Fuge auf unterschiedliche Weise erfolgen. Erfindungswesentlich ist hierbei, dass die Segmente 10 einstückig und ohne Materialverbindung zum einem benachbarten Segment 10 aufgebaut sind, wobei jedoch ihre Anordnung zueinander unverlierbar ist, so dass ihre Trennung nur durch Zerstörung des Grundkörpers der Abwinkelungsvorrichtung erfolgen kann.

Ebenso ist die Verwendung der erfindungsgemäßen Abwinkelungseinrichtung nicht allein auf medizinische Geräte beschränkt, die Erfindung kann vielmehr auch neben der Verwendung allgemein im technischen Bereich wie beispielsweise bei Endoskopen auch für die Einschränkung und/oder Führung einer Abwinkelung und/oder Krümmung zum Beispiel bei Alltagsgegenständen wie Möbel, Lampen etc. eingesetzt werden.

## Patentansprüche

1. Abwinkelungsvorrichtung (100), insbesondere für ein chirurgisches Instrument, mit einem im wesentlichen rohrförmigen, aus zumindest zwei, vorzugsweise aus einer Vielzahl von im wesentlichen ringförmigen Segmenten (10, 10') gebildeten Grundkörper (101) mit einem Innenraum (103), der eine im wesentlichen zentral verlaufende Längsachse (A) aufweist, wobei zumindest ein Segment (10, 10') gegenüber zumindest einem benachbarten Segment (10, 10') über eine in sich geschlossene Fuge (102) von zumindest einem benachbarten Segment (10, 10') beabstandet ist und um eine Schwenkachse (B), die ungleich der Längsachse (A) des Grundkörpers (101) ist, aus einer ersten Position in zumindest eine zweite Position verschwenkbar ist, wobei das zumindest eine Segment (10, 10') unverlierbar an dem benachbarten Segment (10, 10') angeordnet ist, indem das zumindest eine Segment (10, 10') über zumindest zwei diametral zueinander angeordnete Gelenksbereiche (11) verschwenkbar ist, die durch teilweise kreisförmige Vorsprünge (12) gebildet sind, die von einem Grundkörper des Segments (10, 10') vorstehen und die jeweils in einer Aufnahme (13) eines benachbarten Segmentes (10, 10') gelagert sind, **dadurch gekennzeichnet, dass** die Vorsprünge (12) jeweils über eine Materialbrücke (16) mit dem Grundkörper (17) des Segments (10, 10') verbunden sind, die vollständig innerhalb eines Quadranten (18) liegt, der ausgehend vom Mittelpunkt (M) des Vorsprungs (12) einerseits durch eine Parallele (D) zur Längsachse (A) und einer Normalen (N) auf diese Parallele (D) definiert ist.

2. Abwinkelungsvorrichtung (100) nach Anspruch 1, **dadurch gekennzeichnet, dass** das zumindest eine Segment (10, 10') in einem ersten Abschnitt des Grundkörpers zwischen den beiden Gelenksbereichen einen sich in Axialrichtung erstreckenden, rechteckigen ersten Fortsatz (14) und dem ersten Fortsatz (14) gegenüberliegend eine erste Ausnehmung (15) aufweist, die zur Aufnahme des ersten Fortsatzes (14) eines benachbarten Segmentes (10, 10') ausgebildet ist.

3. Abwinkelungsvorrichtung (100) nach Anspruch 2, **dadurch gekennzeichnet, dass** das zumindest eine Segment (10, 10') in einem weiteren Abschnitt des Grundkörpers (17) zwischen den beiden Gelenksbereichen, der dem ersten Abschnitt in Umfangsrichtung gegenüberliegt, eine im Wesentlichen glatte, keilförmige Ausnehmung (26) aufweist.

4. Abwinkelungsvorrichtung (100) nach einem der Ansprüche 1 bis 3, **dadurch gekennzeichnet, dass** ein hakenförmiger Vorsprung (19) eines Segments (10, 10') einen teilweise kreisförmigen Vorsprung (12) des benachbarten Segments (10, 10') mit einem Umfangswinkel (22) von mindestens 90° umgreift.

5. Abwinkelungsvorrichtung (100) nach einem der Ansprüche 2 bis 4, **dadurch gekennzeichnet, dass** die ersten und zweiten Fortsätze (14, 14') entlang einer Linie (C) angeordnet sind, die parallel zu der Längsachse (A) des Grundkörpers (101) verläuft.

6. Abwinkelungsvorrichtung (100) nach Anspruch 2 bis 4, **dadurch gekennzeichnet, dass** die ersten und zweiten Fortsätze (14, 14') in Umfangsrichtung des Grundkörpers (101) versetzt zueinander angeordnet sind.

7. Abwinkelungsvorrichtung (100) nach einem der Ansprüche 1 bis 6, **dadurch gekennzeichnet, dass** das zumindest eine Segment (10, 10') unabhängig von den benachbarten Segmenten (10, 10') verschwenkbar ist.

8. Abwinkelungsvorrichtung (100) nach einem der Ansprüche 1 bis 7, **dadurch gekennzeichnet, dass** die einzelnen Segmente (10, 10') über eine hydraulische, pneumatische oder mechanische Einrichtung bewegbar sind.

9. Abwinkelungsvorrichtung (100) nach einem der Ansprüche 1 bis 8, **dadurch gekennzeichnet, dass** das zumindest eine Segment (10, 10') zumindest eine, vorzugsweise zwei oder mehrere Aufnahmen (105) für Führungselemente, insbesondere Zugseile oder Bowdenzüge aufweist.

10. Medizinisches Instrument mit einem entlang seiner Längsachse krümmbaren länglichen Element, **gekennzeichnet durch** eine Abwinkelungsvorrichtung (100) nach einem der Ansprüche 1 bis 9.

11. Medizinisches Instrument nach Anspruch 10, **dadurch gekennzeichnet, dass** die Abwinkelungsvorrichtung (100) über zumindest ein, vorzugsweise zwei Führungselemente, insbesondere Zugseile oder Bowdenzüge bedienbar ist.

12. Medizinisches Instrument nach Anspruch 11, **dadurch gekennzeichnet, dass** die Abwinkelungsvorrichtung (100) über zumindest ein Hydrauliksystem bedienbar ist.

13. Verfahren zur Herstellung einer Abwinkelungsvorrichtung, insbesondere nach einem der Ansprüche 1 bis 9, **dadurch gekennzeichnet, dass** ein den Grundkörper (101) bildendes Rohr zumindest abschnittsweise in Segmente (10, 10') zerteilt wird, wobei die Segmente (10, 10') voneinander abgetrennt, unlösbar voneinander und gleichzeitig ineinander eingreifend unter Bildung einer in sich geschlossenen Fuge (102) zugeschnitten werden.

14. Verfahren nach Anspruch 13, **dadurch gekennzeichnet, dass** während des Schneidens kleine Stege verbleiben, die die Fuge (102) unterbrechen und in einem weiteren Fertigungsschritt der Abwinkelungsvorrichtung (100) aufgebrochen werden.

15. Verfahren nach Anspruch 13 oder 14, **dadurch gekennzeichnet, dass** die Zerteilung des Rohres mittels Laserschneiden, Wasserstrahlschneiden oder Erosion erfolgt.
